# FASCICULE DE BREVET EUROPEEN

(11) **EP 2 106 269 B1**
(45) Date de publication et mention de la délivrance du brevet: **02.08.2017**
(21) Numéro de dépôt: 07871896.2
(22) Date de dépôt: 10.12.2007
(51) Int. Cl.: A61M 15/00

(54) **DISPOSITIF DE DISTRIBUTION DE PRODUIT FLUIDE**
SPENDER FÜR EIN FLÜSSIGPRODUKT
FLUID PRODUCT DISPENSING DEVICE

(30) Priorité: 11.12.2006 FR 0655411; 03.07.2007 FR 0756238
(43) Date de publication de la demande: 07.10.2009
(73) Titulaire: Aptar France SAS, 27110 Le Neubourg (FR)
(72) Inventeur: SALLAK, Zakaria, 76100 Rouen (FR); KIRNIAK, Maxime, F-08200 Sedan (FR)
(74) Mandataire: CAPRI
(86) Numéro de dépôt international: PCT/FR2007/052464
(87) Numéro de publication internationale: WO 2008/081132

(56) Documents cités:
- WO-A-03/035509
- WO-A-2006/079751
- WO-A-2007/096111
- WO-A-2008/012458

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs pourvus de bande de blisters est lié au déplacement de la bande, et au stockage de la partie utilisée de la bande. Ainsi, selon la longueur de la bande, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. WO2006/079751 A1 divulgue les caractéristiques du préambule de la revendication 1. La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel inhalateur pourvu d'une bande de blisters, dans lequel le stockage de la partie de bande utilisée est optimisé, et le risque de blocage de la bande minimisé.

La présente invention a donc pour objet un dispositif de distribution de produit fluide, comportant un corps, ledit dispositif comportant en outre une bande souple allongée supportant une pluralité de réservoirs contenant chacun une dose de produit fluide ou pulvérulent, des moyens d'ouverture de réservoir pour ouvrir un réservoir respectif à chaque actionnement, des premiers moyens de déplacement pour faire avancer ladite bande souple avant et/ou pendant et/ou après chaque actionnement, pour amener un réservoir plein face auxdits moyens d'ouverture de réservoir et des seconds moyens de déplacement, pour déplacer un réservoir plein contre lesdits moyens d'ouverture à chaque actionnement du dispositif, l'extrémité avant de ladite bande souple, dans le sens d'avancement de celle-ci, étant fixée à un élément de réception monté rotatif, ledit élément de réception étant fixé à un ressort chargé adapté à exercer une force sur ledit élément de réception pour le solliciter en rotation, de sorte que ledit élément de réception exerce une force de traction sur ladite bande allongée, ladite force de traction étant indépendante desdits premiers et seconds moyens de déplacement.

Avantageusement, ladite force de traction est maximale en début d'utilisation du dispositif et diminuant à chaque actionnement, au fur et à mesure que le ressort se décharge.

Avantageusement, ledit ressort est un ressort en spirale, un ressort à lame ou un ressort hélicoïdal.

Avantageusement, ledit ressort est d'une part fixé à un axe de fixation fixe en rotation, et d'autre part audit élément de réception rotatif.

Avantageusement, ledit élément de réception comporte un premier organe de fixation auquel est fixée la bande.

Avantageusement, ledit premier organe de fixation forme une chicane pour la bande.

Avantageusement, l'extrémité avant de la bande forme une boucle autour dudit premier organe de fixation.

Avantageusement, ledit élément de réception comporte un second organe de fixation auquel est fixée une partie du ressort.

Avantageusement, ledit élément de réception est encliqueté sur une surface de support, de préférence au moyen d'ergots d'encliquetage, pour fixer axialement et transversalement ledit élément de réception, tout en lui permettant de tourner autour d'un axe de fixation fixe en rotation auquel est fixée une partie du ressort.

Avantageusement, ledit élément de réception est fixé de manière rotative sur une surface de support solidaire desdits seconds moyens de déplacement, ledit élément de support étant donc déplacé à chaque actionnement du dispositif ensemble avec le réservoir à ouvrir.

Avantageusement, lesdits moyens d'ouverture comportent une aiguille fixe par rapport audit corps, un réservoir étant déplacé contre ladite aiguille à chaque actionnement du dispositif, ladite aiguille pénétrant dans ledit réservoir pour le vider au moyen de l'écoulement d'inhalation.

Avantageusement, lesdits moyens d'ouverture sont commandés par l'inhalation de l'utilisateur, de sorte que le réservoir est simultanément ouvert et vidé par ledit écoulement d'inhalation.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 représente une vue schématique partielle en perspective d'un dispositif de distribution selon un mode de réalisation avantageux de l'invention,
- la figure 2 est une vue en section transversale du dispositif de la figure 1, en début d'utilisation, avec une majorité de réservoirs pleins,
- la figure 3 est une vue similaire à celle de la figure 2, après plusieurs utilisations, avec une majorité de réservoirs vides, et
- les figures 4 à 8 montrent schématiquement des variantes de réalisation de l'élément de réception.

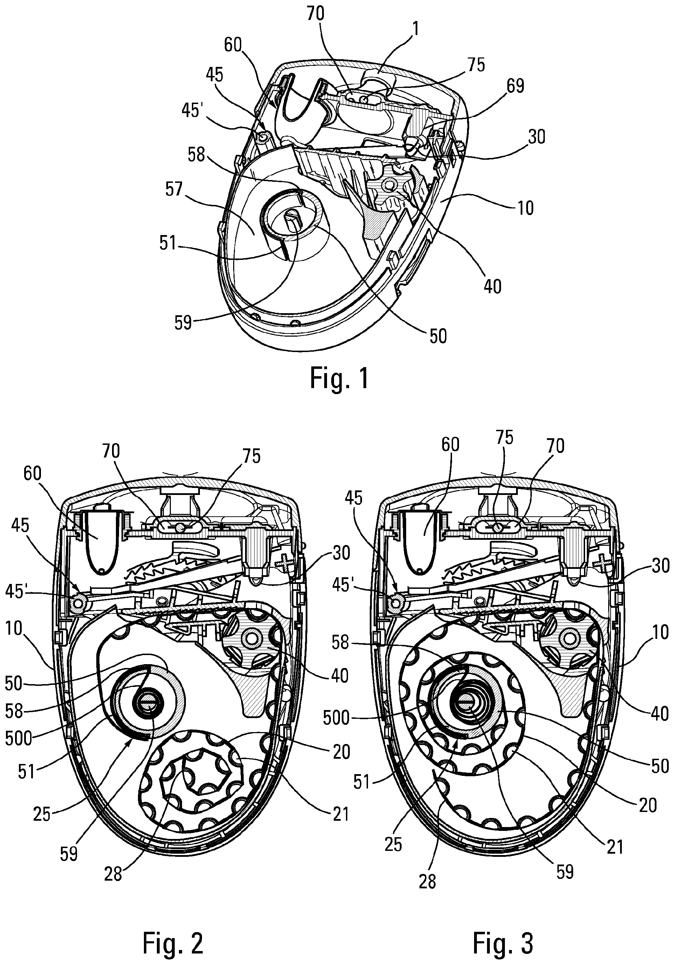

Sur les figures 1 à 3 est représentée une variante de réalisation avantageuse d'un inhalateur de poudre sèche. Cet inhalateur comporte un corps 10 sur lequel peuvent être montées coulissantes deux parties formant capot (non représentées) adaptées à être ouvertes pour ouvrir et charger le dispositif. Le corps 10 peut être de forme environ arrondie comme représenté sur les figures, mais il pourrait avoir tout autre forme appropriée. Le corps 10 comporte un embout buccal ou d'inhalation 1 définissant un orifice de distribution à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable. En variante, le dispositif pourrait comporter un seul capot au lieu de deux.

A l'intérieur du corps 10, il est prévu une bande 20 de réservoirs individuels 21, également appelés blisters, réalisée sous forme d'une bande allongée 20 sur laquelle les blisters 21 sont disposés les uns derrière les autres, de manière connue. Ces blisters 21, contenant de préférence de la poudre, ne sont pas représentés sur la figure 1 pour ne pas surcharger le dessin dans des buts de clarté. Cette bande de blister 20 est avantageusement constituée d'une couche ou paroi de base formant les cavités recevant les doses de poudre, et d'une couche ou paroi de fermeture qui obture de manière étanche chacun desdits blisters 21. Avant la première utilisation, la bande de blister 20 peut être enroulée à l'intérieur du corps 10, de préférence dans une partie de stockage, et des premiers moyens de déplacement de bande 40 sont prévus pour progressivement dérouler et faire avancer cette bande de blister. Des seconds moyens de déplacement 45 sont prévus pour amener un réservoir individuel ou blister respectif 21 dans une position de distribution à chaque actionnement du dispositif. La partie de bande comportant les réservoirs vides est avantageusement adaptée à s'enrouler dans un autre endroit dudit corps 10, de préférence une partie de réception, comme cela sera décrit plus en détail ci-après.

L'inhalateur comporte des moyens d'ouverture de réservoir 30 comportant de préférence des moyens de perçage et/ou de coupage de la couche de fermeture des blisters. Par exemple, les moyens d'ouverture de réservoir comportent avantageusement une aiguille 30, de préférence fixe par rapport au corps 10, et contre laquelle un blister respectif 21 est déplacé à chaque actionnement par les seconds moyens de déplacement 45. Le blister est alors percé par ladite aiguille, qui pénètre dans ledit blister pour expulser la poudre au moyen du flux d'inhalation de l'utilisateur.

Les premiers moyens de déplacement 40 sont adaptés à faire avancer la bande de blisters 20 avant et/ou pendant et/ou après chaque actionnement du dispositif. Les seconds moyens de déplacement 45 sont adaptés à déplacer le réservoir à vider 21 contre lesdits moyens de perçage et/ou de coupage 30 lors de l'actionnement. Ces seconds moyens de déplacement 45 peuvent être sollicités par un élément élastique, tel qu'un ressort ou tout autre élément élastique équivalent, ledit élément élastique pouvant être préchargé lors de l'ouverture du dispositif. De préférence, les premiers moyens de déplacement 40 comportent une roue d'indexage 40 qui reçoit et guide les blisters. Une rotation de cette roue 40 fait avancer la bande de blister 20. Dans une position angulaire particulière, un réservoir donné 21 est toujours en position face aux moyens d'ouverture 30. Les seconds moyens de déplacement 45 peuvent comporter un élément de support rotatif autour d'un axe de rotation, ladite roue d'indexage 40 étant montée rotative sur ledit élément de support.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage 40 ne peut pas se déplacer vers l'aiguille 30 car les seconds moyens de déplacement 45 sont retenus par des moyens de blocage appropriés. De préférence, c'est lors de l'inhalation par l'utilisateur à travers l'embout buccal 1 que ces moyens de blocage sont débloqués, ce qui provoque alors le déplacement de ladite roue d'indexage 40 en direction de l'aiguille 30, et donc l'ouverture d'un réservoir 21.

Dans les exemples représentés, le réservoir 21 se déplace vers sa position d'ouverture pour être ouvert par l'aiguille 30 qui est fixe par rapport au corps 10. Toutefois, il est envisageable que cette aiguille puisse également être mobile pendant la phase d'ouverture du réservoir 21. Par exemple, l'aiguille pourrait se déplacer en direction du réservoir 21 pendant que le réservoir 21 se déplace en direction de l'aiguille. Dans une autre variante, on pourrait également envisager que le réservoir 21 et l'aiguille se déplacent dans la même direction lors de l'actionnement, le réservoir 21 se déplaçant plus rapidement dans cette direction de sorte qu'il vient en contact avec l'aiguille pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement une unité 60 déplaçable et/ou déformable sous l'effet de l'inhalation, cette unité 60 étant adaptée à libérer les moyens de blocage. Cette unité 60 comprend avantageusement une chambre d'air déformable. L'inhalation de l'utilisateur provoque la déformation de ladite chambre d'air déformable, permettant ainsi de libérer lesdits moyens de blocage et donc de permettre le déplacement des seconds moyens de déplacement 45, et donc d'un réservoir respectif 21 vers sa position d'ouverture. Le réservoir 21 n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

L'inhalateur comporte en outre une chambre de distribution 70 qui est destinée à recevoir la dose de poudre après ouverture d'un réservoir respectif 21. Avantageusement, cette chambre de distribution 70 est pourvue d'au moins une bille 75 qui se déplace à l'intérieur de ladite chambre 70 pendant l'inhalation, pour améliorer la distribution du mélange air et poudre après ouverture d'un réservoir 21, afin d'augmenter l'efficacité du dispositif.

Il peut être avantageux que les moyens d'ouverture 30, en particulier l'aiguille, soient formés directement sur ladite chambre de distribution 70, par exemple à l'extrémité d'un canal 69 menant à ladite chambre 70.

Après l'inhalation, lorsque l'utilisateur referme le dispositif, tous les composants reviennent vers leur position de repos initiale. Le dispositif est alors prêt pour un nouveau cycle d'utilisation.

Selon un aspect avantageux de l'inhalateur, les réservoirs individuels ou blisters 21 sont formés sur une bande allongée 20, qui, au début, est principalement stockée sous forme de bobine dans un logement de stockage à l'intérieur du corps 10 du dispositif. Avantageusement, cette bande de blister enroulée 20 est maintenue par des parois internes dudit logement de stockage sans que son extrémité arrière 28 (dans le sens d'avancement de la bande de blisters 20) ne soit fixée par rapport audit corps 10, ce qui permet un assemblage plus aisé de cette bobine de bande de blister à l'intérieur du dispositif. La bande de blister 20 est déplacée par l'utilisateur avantageusement au moyen de la roue d'indexage 40 qui présente avantageusement au moins un, de préférence plusieurs évidements 41, dont la forme correspond à celle des blisters. Ainsi, lorsque cette roue d'indexage 40 tourne, elle fait avancer la bande de blister 20. Bien entendu en variante ou de manière additionnelle, on pourrait utiliser d'autres moyens d'avancée de bande de blisters, par exemple en prévoyant un profil sur les bords longitudinaux latéraux de la bande de blister ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous.

Après ouverture d'un ou plusieurs blister(s), la partie de bande de blister avec les réservoirs vidés doit pouvoir être stockée de manière aisée et non encombrante dans le dispositif, en évitant tout risque de blocage. Avantageusement, cette bande de blister usée s'enroule automatiquement sur elle-même pour à nouveau former une bobine.

Selon l'invention, l'extrémité avant 25 de cette bande de blister 20 est fixé à un élément de réception 50 monté rotatif sur une surface de support 57. Cette surface de support 57 est de préférence mobile sous l'effet des seconds moyens de déplacement 45, c'est-à-dire que l'élément de réception 50 se déplace avec le réservoir à vider à chaque actionnement. Dans l'exemple des figures 1 à 3, c'est l'ensemble de la cassette recevant la bande 20, la roue d'indexage 40 et la surface de support 57 qui pivote autour de l'axe de pivotement 45'. Pour assurer un bon enroulement de la partie avant de la bande de blisters 20, à savoir celle comportant les blisters vides, dans la partie de réception, cet élément de réception rotatif 50 est adapté à exercer une force de traction sur la bande 20, en particulier sur son extrémité avant 25. Ainsi, on évite tout risque de mauvais enroulement, par exemple de pliage en accordéon ou similaire, de la bande, qui risquerait de bloquer le dispositif. Cette force de traction est exercée par un ressort 500, qui sollicite ledit élément de réception 50 en rotation, et donc tire sur la bande. Ce ressort peut notamment être un ressort à spirale, une ressort à lame ou un ressort hélicoïdal.

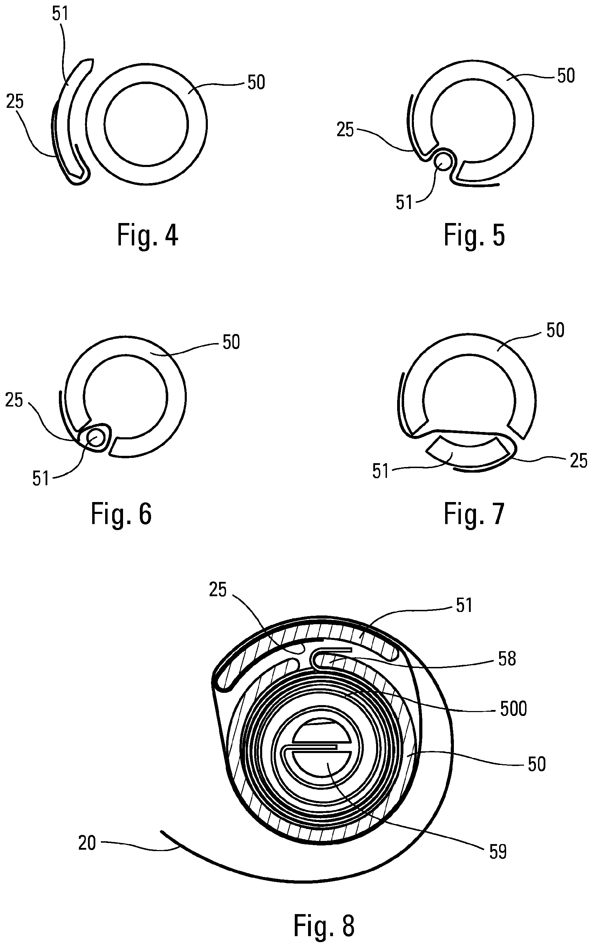

Les figures 1 à 3 montrent une variante de réalisation avantageuse, dans laquelle l'élément de réception 50 forme un cylindre monté rotatif autour d'un axe de fixation 59 fixe en rotation et auquel est fixée une partie, de préférence une extrémité, du ressort 500. Ainsi, le ressort 500 est ici disposé autour dudit axe de fixation 59, à l'intérieur dudit cylindre formant l'élément de réception 50. Avantageusement, l'extrémité avant 25 de la bande de blisters 20 est fixée à un premier organe de fixation 51, qui peut former une chicane pour coincer la bande 20. Différentes variantes sont représentées sur les figures 4 à 8. Sur les figures 4, 7 et 8, l'extrémité avant 25 de la bande 20 est recourbée autour d'un coin 51 de l'élément de réception 50, alors que sur la figure 6, elle fait une boucle autour d'un plot 51. L'important étant d'assurer une fixation suffisante pour résister à la force du ressort 500, maximale au début, et qui diminue progressivement. L'autre extrémité du ressort 500 peut être fixée à un second organe de fixation 58 de l'élément de réception 50, pouvant également former une chicane pour une partie du ressort, de préférence son autre extrémité, comme visible sur les figures 2, 3 et 8.

Le ressort 500 est chargé avant la première utilisation ou lors de l'assemblage, et de manière évidente, la force de traction maximale exercée sur la bande 20 est insuffisante pour déchirer, déformer ou déplacer la bande 20 en l'absence d'actionnement. Progressivement, à chaque actionnement, le ressort se décharge en faisant tourner l'élément de réception 50. Les caractéristiques du ressort 500 sont de préférence choisies de telle sorte qu'il exerce une force de traction jusqu'aux dernières doses, mais dans certaines applications, il peut être suffisant que cette force de traction ne soit exercée qu'en début d'utilisation, pour garantir un bon début d'enroulement de la partie de bande avec les réservoirs vides.

L'élément de réception 50 est de préférence fixé sur la surface de support 57, notamment encliqueté, de telle sorte qu'il peut se déplacer seulement en rotation, mais ni latéralement (ou transversalement), ni verticalement (ou axialement). Avantageusement, l'encliquetage peut se faire au moyen d'ergots d'encliquetage pour limiter les frottements lors de la rotation.

La force de traction exercée par l'élément rotatif 50 sur la bande 20 est totalement indépendante des premiers moyens de déplacement, à savoir la roue d'indexage 40 qui fait avancer la bande lors de chaque actionnement. Ceci permet de garantir que la force de traction ne sera pas dépendante du diamètre d'enroulement de la bande de blisters usée, comme cela serait le cas si la rotation de l'élément rotatif de réception 50 était corrélée à celle de la roue d'indexage 40. Elle est aussi totalement indépendante des seconds moyens de déplacement 45, de sorte que l'invention évite de prévoir des moyens d'actionnement relativement complexes pour créer une force de traction sur la bande lors de l'actionnement de l'inhalateur. Ceci simplifie la fabrication et l'assemblage de l'inhalateur.

Avantageusement, l'élément de réception 50 est disposé environ au centre de la partie de réception. Celle-ci peut comporter des parois de guidage, en particulier une paroi de guidage externe, courbée, par exemple cylindrique, contre laquelle va glisser la bande de blisters 20. Il peut aussi y avoir une paroi de guidage interne prévue à l'entrée de la partie de réception, et s'étendant de préférence environ parallèlement à la paroi de guidage externe pour former un canal de guidage pour la bande de blisters 20. Ces parois de guidage favorisent encore davantage un bon enroulement de la bande de blisters 20 autour de l'élément de réception 50.

La présente invention permet donc de fournir un inhalateur de poudre sèche qui procure notamment les fonctions suivantes :
▪ une pluralité de doses individuelles de poudre stockées dans des réservoirs individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ un stockage sûr et fiable de la partie de la bande usée, par enroulement autour d'un élément rotatif adapté à tirer sur la bande à chaque actionnement, cette traction étant totalement indépendante des premiers moyens de déplacement, à savoir de la roue d'indexage 40 servant à faire avancer la bande de blisters 20.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées.

## Revendications

1. Dispositif de distribution de produit fluide, comportant un corps (10), ledit dispositif comportant en outre une bande souple allongée (20) supportant une pluralité de réservoirs (21) contenant chacun une dose de produit fluide ou pulvérulent, des moyens d'ouverture de réservoir (30) pour ouvrir un réservoir respectif à chaque actionnement, des premiers moyens de déplacement (40) pour faire avancer ladite bande souple (20) avant et/ou pendant et/ou après chaque actionnement, pour amener un réservoir plein face auxdits moyens d'ouverture de réservoir et des seconds moyens de déplacement (45), pour déplacer un réservoir plein (21) contre lesdits moyens d'ouverture (30) à chaque actionnement du dispositif, **caractérisé en ce que** l'extrémité avant (25) de ladite bande souple (20), dans le sens d'avancement de celle-ci, est fixée à un élément de réception (50) monté rotatif, ledit élément de réception (50) étant fixé à un ressort chargé (500) adapté à exercer une force sur ledit élément de réception (50) pour le solliciter en rotation, de sorte que ledit élément de réception (50) exerce une force de traction sur ladite bande allongée (20), ladite force de traction étant indépendante desdits premiers et seconds moyens de déplacement (40, 45).

2. Dispositif selon la revendication 1, dans lequel ladite force de traction est maximale en début d'utilisation du dispositif et diminuant à chaque actionnement, au fur et à mesure que le ressort (500) se décharge.

3. Dispositif selon la revendication 1 ou 2, dans lequel ledit ressort (500) est un ressort en spirale, un ressort à lame ou un ressort hélicoïdal.

4. Dispositif selon l'une quelconque des revendications précédentes, dans lequel ledit ressort est d'une part fixé à un axe de fixation (59) fixe en rotation, et d'autre part audit élément de réception rotatif (50).

5. Dispositif selon l'une quelconque des revendications précédentes, dans lequel ledit élément de réception (50) comporte un premier organe de fixation (51) auquel est fixée la bande (20).

6. Dispositif selon la revendication 5, dans lequel ledit premier organe de fixation (51) forme une chicane pour la bande (20).

7. Dispositif selon la revendication 5 ou 6, dans lequel l'extrémité avant (25) de la bande (20) forme une boucle autour dudit premier organe de fixation (51).

8. Dispositif selon l'une quelconque des revendications précédentes, dans lequel ledit élément de réception (50) comporte un second organe de fixation (58) auquel est fixée une partie du ressort (500).

9. Dispositif selon l'une quelconque des revendications précédentes, dans lequel ledit élément de réception (50) est encliqueté sur une surface de support (57), de préférence au moyen d'ergots d'encliquetage, pour fixer axialement et transversalement ledit élément de réception (50), tout en lui permettant de tourner autour d'un axe de fixation (59) fixe en rotation auquel est fixée une partie du ressort (500).

10. Dispositif selon l'une quelconque des revendications précédentes, dans lequel ledit élément de réception (50) est fixé de manière rotative sur une surface de support (57) solidaire desdits seconds moyens de déplacement (45), ledit élément de support (50) étant donc déplacé à chaque actionnement du dispositif ensemble avec le réservoir à ouvrir (21).

11. Dispositif selon l'une quelconque des revendications précédentes, dans lequel lesdits moyens d'ouverture (30) comportent une aiguille (30) fixe par rapport audit corps (10), un réservoir (21) étant déplacé contre ladite aiguille à chaque actionnement du dispositif, ladite aiguille pénétrant dans ledit réservoir (21) pour le vider au moyen de l'écoulement d'inhalation.

12. Dispositif selon l'une quelconque des revendications précédentes, dans lequel lesdits moyens d'ouverture (30) sont commandés par l'inhalation de l'utilisateur, de sorte que le réservoir (21) est simultanément ouvert et vidé par ledit écoulement d'inhalation.

## Patentansprüche

1. Ausgabevorrichtung für ein fluides Produkt, aufweisend einen Körper (10), wobei die Vorrichtung des Weiteren einen flexiblen, langgestreckten Streifen (20), der mehrere Behälter (21) trägt, die jeweils eine Dosis von fluidem oder pulverförmigem Produkt enthalten, Behälteröffnungsmittel (30), um bei jeder Betätigung jeweils einen Behälter zu öffnen, erste Verschiebemittel (40), um den flexiblen Streifen (20) vor und/oder während und/oder nach jeder Betätigung vorwärts zu bewegen, um einen vollen Behälter in Position gegenüber den Behälteröffnungsmitteln zu bringen, und zweite Verschiebemittel (45) aufweist, um bei jeder Betätigung der Vorrichtung einen vollen Behälter (21) in Position gegen die Öffnungsmittel (30) zu verschieben, **dadurch gekennzeichnet, dass** das vordere Ende (25) des flexiblen Streifens (20) in Vorschubrichtung von diesem an einem drehbar gelagerten Aufnahmeelement (50) befestigt ist, wobei das Aufnahmeelement (50) an einer gespannten Feder (500) befestigt ist, die dafür geeignet ist, auf das Aufnahmeelement (50) eine Kraft auszuüben, um dieses drehend zu belasten, so dass das Aufnahmeelement (50) auf den langgestreckten Streifen (20) eine Zugkraft ausübt, wobei die Zugkraft von den ersten und zweiten Verschiebemitteln (40, 45) unabhängig ist.

2. Vorrichtung nach Anspruch 1, wobei die Zugkraft zu Beginn der Verwendung der Vorrichtung maximal ist und bei jeder Betätigung in dem Maße, wie die Feder (500) entlastet wird, abnimmt.

3. Vorrichtung nach Anspruch 1 oder 2, wobei die Feder (500) eine Spiralfeder, eine Blattfeder oder eine Schraubenfeder ist.

4. Vorrichtung nach einem der vorhergehenden Ansprüche, wobei die Feder einerseits an einer drehfesten Befestigungsachse (59) und andererseits an dem drehbaren Aufnahmeelement (50) befestigt ist.

5. Vorrichtung nach einem der vorhergehenden Ansprüche, wobei das Aufnahmeelement (50) eine erste Befestigungseinrichtung (51) aufweist, an der der Streifen (20) befestigt ist.

6. Vorrichtung nach Anspruch 5, wobei die erste Befestigungseinrichtung (51) ein Hindernis für den Streifen (20) bildet.

7. Vorrichtung nach Anspruch 5 oder 6, wobei das vordere Ende (25) des Streifens (20) eine Schleife um die erste Befestigungseinrichtung (51) bildet.

8. Vorrichtung nach einem der vorhergehenden Ansprüche, wobei das Aufnahmeelement (50) eine zweite Befestigungseinrichtung (58) aufweist, an der ein Teil der Feder (500) befestigt ist.

9. Vorrichtung nach einem der vorhergehenden Ansprüche, wobei das Aufnahmeelement (50) vorzugsweise mittels Einrastnocken auf einer Stützfläche (57) eingerastet ist, um das Aufnahmeelement (50) axial und quer zu befestigen, wobei es sich gleichzeitig um eine drehfeste Befestigungsachse (59) drehen kann, an der ein Teil der Feder (500) befestigt ist.

10. Vorrichtung nach einem der vorhergehenden Ansprüche, wobei das Aufnahmeelement (50) drehend auf einer Stützfläche (57) in einem Stück mit den zweiten Verschiebemitteln (45) befestigt ist, wobei das Stützelement (50) dann bei jeder Betätigung der Vorrichtung zusammen mit dem zu öffnenden Behälter (21) verschoben wird.

11. Vorrichtung nach einem der vorhergehenden Ansprüche, wobei die Öffnungsmittel (30) eine Nadel (30) aufweisen, die in Bezug auf den Körper (10) befestigt ist, wobei ein Behälter (21) bei jeder Betätigung der Vorrichtung gegen die Nadel verschoben wird, wobei die Nadel in den Behälter (21) eindringt, um ihn mittels Inhalationsströmung zu leeren.

12. Vorrichtung nach einem der vorhergehenden Ansprüche, wobei die Öffnungsmittel (30) durch Inhalation des Benutzers gesteuert werden, so dass der Behälter (21) durch die Inhalationsströmung gleichzeitig geöffnet und geleert wird.

## Claims

1. A fluid dispenser device comprising a body (10), said device further comprising: an elongate flexible strip (20) supporting a plurality of reservoirs (21) each containing a dose of fluid or powder; reservoir-opening means (30) for opening a respective reservoir on each actuation; first displacement means (40) for causing said flexible strip (20) to advance before and/or during and/or after each actuation, so as to bring a full reservoir into register with said reservoir-opening means; and second displacement means (45) for displacing a full reservoir (21) against said opening means (30) each time the device is actuated, said device being **characterized in that** the leading end (25) of said flexible strip (20), in the advance direction of said strip, is fastened to a receiver element (50) that is rotatably mounted, said receiver element (50) being fastened to a loaded spring (500) that is adapted to exert a force on said receiver element (50) so as to urge it to turn, such that said receiver (50) exerts a traction force on said elongate strip (20), said traction force being independent of said first and second displacement means (40, 45).

2. A device according to claim 1, in which said traction force is at a maximum when the device is first used and reduces on each actuation as the spring (500) relaxes.

3. A device according to claim 1 or claim 2, in which said spring (500) is a spiral spring, a leaf spring, or a helical spring.

4. A device according to any preceding claim, in which said spring is fastened firstly to a fastener pin (59) that cannot turn, and secondly to said rotary receiver element (50).

5. A device according to any preceding claim, in which said receiver element (50) includes a first fastener member (51) to which there is fastened the strip (20).

6. A device according to claim 5, in which said first fastener member (51) forms a baffle for the strip (20).

7. A device according to claim 5 or claim 6, in which the leading end (25) of the strip (20) forms a loop around said first fastener member (51).

8. A device according to any preceding claim, in which said receiver element (50) includes a second fastener member (58) to which there is fastened a portion of the spring (500).

9. A device according to any preceding claim, in which said receiver element (50) is snap-fastened on a support surface (57), preferably by means of snap-fastener studs, so as to fasten said receiver element (50) both axially and transversally, while enabling it to turn about a fastener pin (59) that cannot turn, and to which there is fastened a portion of the spring (500).

10. A device according to any preceding claim, in which said receiver element (50) is rotatably fastened on a support surface (57) that is secured to said second displacement means (45), said support element (50) thus being displaced each time the device is actuated, together with the reservoir (21) to be opened.

11. A device according to any preceding claim, in which said opening means (30) comprise a needle (30) that does not move relative to said body (10), a reservoir (21) being displaced against said needle each time the device is actuated, said needle penetrating into said reservoir (21) so as to empty it by means of an inhalation flow.

12. A device according to any preceding claim, in which said opening means (30) are controlled by the user inhaling, such that the reservoir (21) is opened and emptied simultaneously by said inhalation flow.
